# EUROPÄISCHE PATENTSCHRIFT

(11) **EP 0 589 235 B1**
(45) Veröffentlichungstag und Bekanntmachung des Hinweises auf die Patenterteilung: **02.06.1999**
(21) Anmeldenummer: 93113711.1
(22) Anmeldetag: 27.08.1993
(51) Int. Cl.: A61B 17/68

(54) **Marknagel mit Arretierungsbolzen**
Intramedullary nail with locking pin
Clou centromedullaire avec boulon de verrouillage.

(30) Priorität: 23.09.1992 CH 2971/92
(43) Veröffentlichungstag der Anmeldung: 30.03.1994
(73) Patentinhaber: Kropf, Philipp Rolf, CH-8142 Uitikon (CH); Geisser, Albert, CH-6373 Ennetbürgen (CH)
(72) Erfinder: Kropf, Philipp Rolf, CH-8142 Uitikon (CH); Geisser, Albert, CH-6373 Ennetbürgen (CH)
(74) Vertreter: Blum, Rudolf Emil Ernst

(56) Entgegenhaltungen:
- AT-B- 384 359
- DE-A- 3 138 311
- DE-A- 3 933 217
- FR-A- 948 690
- US-A- 3 741 205

## Beschreibung

Die Erfindung beschreibt einen Marknagel mit Arretierungsbolzen gemäss Oberbegriff von Anspruch 1. Ein derartiger Marknagel wird in AT-B-384 359 beschrieben, wobei bei dieser Lösung ein guter Halt der Arretierungsbolzen nicht gewährleistet ist.

Bei chirurgischen Eingriffen zur Behebung von Knochenschäden werden oftmals Schrauben oder speziell ausgebildete Nägel verwendet, um Stabilisierungshilfen, wie z. B. Osteosyntheseplatten, Gelenkprothesen, Marknägel und dergleichen, mit dem Knochen zu verbinden oder um Knochenfragmente untereinander zu stabilisieren. Typische Schrauben und Nägel dieser Art werden z. B. in den Schweizer Patentschriften 670 754 und 669 724 beschrieben.

Die dabei verwendeten Schrauben und Nägel weisen in der Regel mindestens über einen Teil ihres Schaftes ein Gewinde oder widerhakenartige Ausformungen auf, mit denen sie gegen ein allfälliges Zurückrutschen gesichert werden sollen. Diese unregelmässige Oberflächenform führt aber in vielen Fällen zu Problemen. Werden solche Schrauben und Nägel statisch oder dynamisch belastet, so kommt es zu einer ungleichmässigen Druckverteilung im Knochengewebe. So können im Bereich von Gewindekanten und Widerhaken hohe Druckspitzen entstehen. Diese können eine Resorption, d.h. einen Abbau, des Knochenmaterials bewirken. Dies führt zu einer Schwächung des Knochens und einer Lockerung der Schraube resp. des Nagels, was zu ungünstiger Kraftverteilung und Brüchen im Knochen und in der Schraube resp. dem Nagel führen kann.

Wird zum Beispiel eine Schraube durch einen Röhrenknochen geführt und mit ihrem Ende in der dem Kopf gegenüberliegenden Kortikalis verschraubt, so kann der beschriebene Effekt zu einer Lockerung des verschraubten Endes führen, wodurch zusätzliche Kräfte auf den Kopfbereich der Schraube verlagert werden. Dies kann einen Bruch der Schraube oder eine Beschädigung des Knochens im Kopfbereich der Schraube bewirken.

Auch sind die herkömmlichen Schrauben nicht rotationsstabil, d.h. sie können verdreht und somit gelockert werden, was wiederum zu zusätzlichen Kräften und zu ungleichmässiger Kraftverteilung und somit zu möglichen Beschädigungen von Schrauben und Knochen führt.

In DE-A-39 33 217 wird eine Knochenschiene mit Arretierungsbolzen gezeigt, wobei die Arretierungsbolzen spreizbar sind. Solche Bolzen besitzen jedoch Längsschlitze, die ebenfalls zu einer ungleichen Kraftverteilung führen können.

Ein weiterer Nachteil herkömmlicher Lösungen liegt darin, dass in der Regel Löcher im Knochen vorgebohrt und oft sogar Gewinde geschnitten werden müssen. Dies bringt einen zusätzlichen Arbeitsgang mit sich, der den Eingriff verteuert. Ausserdem geht dabei Knochenmaterial verloren, was zu einer weiteren Schwächung des Knochens führt.

Deshalb stellt sich die Aufgabe, einen Marknagel mit Arretierungsbolzen der eingangs genannten Art bereitzustellen, der die genannten Nachteile möglichst nicht aufweist und einen guten Halt des Bolzens im Marknagel gewährleistet.

Diese Aufgabe wird durch den im ersten Patentanspruch beschriebenen Gegenstand gelöst.

Da der erfindungsgemässe Bolzen eine im wesentlichen glatte Oberfläche hat, die insbesondere kein Gewinde und keine Widerhaken aufweist, bewirkt er keine ungleichmässige Druckverteilung im Knochengewebe. Damit wird die Resorption des Knochenmaterials verhindert.

Ausserdem kann der Bolzen direkt ohne Vorbohrung in den Knochen eingebracht werden, was die Operationszeit erheblich verkürzt und den Verlust an Knochenmaterial verkleinert.

Der erfindungsgemässe Bolzen ist auch dafür geeignet, mit einem pneumatischen Schlagwerkzeug eingeschlagen zu werden. In diesem Fall wird die Belastung des Knochens und insbesondere die Gefahr von Knochenrissen während des Einschlagens stark verringert. Derart eingebrachte Bolzen zeichnen sich durch einen ausgezeichneten Halt im Knochen aus.

Weitere Vorteile ergeben sich aus der folgenden Beschreibung anhand der Zeichnungen. Dabei zeigen:
Figur 1 die Seitenansicht eines gebogenen Marknagels und einen Schnitt durch und eine Aufsicht auf einen Marknagel mit speziell der Erfindung angepassten Fixierungslöchern;
Figur 2 einen Bolzen, dessen Schaftdurchmesser nach Einbringen in den Knochen aufweitbar ist; und
Figur 3 den Bolzen aus Figur 2 mit eingesetztem Andruckelement.

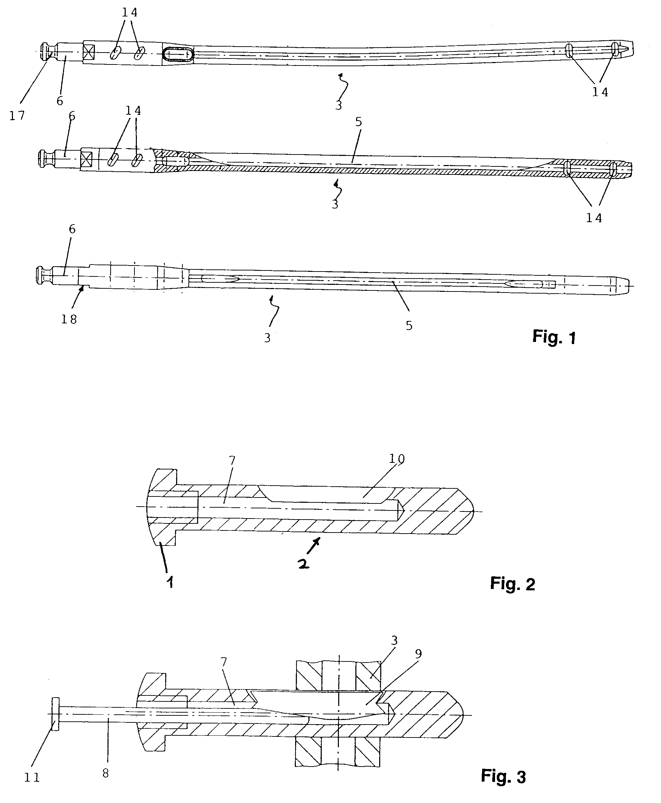

Figur 1 zeigt einen Marknagel 3. Er weist Löcher 14 zur Aufnahme des Schafts eines Bolzens auf, welche dessen Querschnittsform angepasst sind.

Der Marknagel nach Figur 1 ist so ausgestaltet, dass er mit einem pneumatischen Schlagwerkzeug direkt in den Knochen eingebracht werden kann, wie es in EP 452 543 beschrieben ist. Insbesondere weist der Marknagel 3 eine makroskopisch im wesentlichen glatte Oberfläche und eine Abflussausformung 5 zum Ableiten des Knochenmarks beim Einbringvorgang auf. Diese ist hier in Form einer ausgefrästen Längsrinne mit Abflusskanälen ausgeführt. Auch ist sein Kopf 6 zur kraftschlüssigen Verbindung mit dem Einschlagwerkzeug ausgeformt. Dazu weist der Kopf 6 eine umgehende Rille 17 auf, in welche das Einschlagwerkzeug eingerastet wird. Damit ist die resultierende Verbindung geeignet, sowohl Zug- wie auch Druckkräfte zu übertragen. Ausserdem weist der Kopf 6 zwei gegenüberliegende Flächen 18 auf, an denen je ein entsprechendes Gegenstück des aufgesetzten Einschlaginstruments anliegt. Somit kann sich der Marknagel gegen das Einschlaginstrument nicht verdrehen.

Durch eine derartige zug-, stoss- und rotationsfeste Verbindung zwischen Marknagel und Einschlaginstrument wird die Kontrolle beim Einschlagen und Herausziehen verbessert.

Dank der Verwendung des pneumatischen Schlagwerkzeuges kann eine Beschädigung des Knochens vermieden werden, selbst wenn der Marknagel einen relativ grossen Durchmesser aufweist. Somit können auch Marknägel verwendet werden, die im eingesetzten Zustand an der Kortikalis eng anliegen.

Da die Positionierung eines Marknagels und der ihn fixierenden Bolzen (oder herkömmlichen Schrauben) bekanntlich nicht beliebig genau erfolgen kann, ist es notwendig, dass die Befestigungslöcher 14 des Marknagels etwas grösser sind als der Schaft der Bolzen. Damit ist es möglich, Toleranzen in den Positionierungen der Elemente auszugleichen. Allerdings bewirkt dies, dass die Bolzen nicht oder nur teilweise in kraftschlüssigem Kontakt mit dem Marknagel stehen.

Zur Behebung dieses Problems kann die Ausführung des erfindungsgemässen Bolzens verwendet werden, die in Figur 2 und 3 gezeigt wird. Dieser Bolzen weist eine Längsbohrung 7 zur Aufnahme eines Stiftes 8 auf. Ausserdem ist ein Teil der Schaftwand durch ein Andruckelement 9 ersetzt, das in der Aussparung 10 liegt. Das Andruckelement ist so geformt, dass es zum Teil in die Längbohrung 7 hineinragt. Wird der Stift 8 in die Längsbohrung 7 eingebracht, so drängt er das Ahdruckelement gegen aussen. Dazu ist das Andruckelement elastisch ausgeformt und/oder beweglich gelagert.

Beim Einbringen des Bolzens in den Knochen ist der Stift 8 nicht eingesetzt. Damit liegt das Andruckelement formschlüssig zur Schaftwand im Bolzen, wodurch der Bolzen wie ein gewöhnlicher erfindungsgemässer Bolzen eingeschlagen werden kann. Die Position des Andruckelements 9 ist so gewählt, dass es nach dem Einbringen im Bereich des Marknagels 3 zu liegen kommt. Wird jetzt der Stift 8 in die Bohrung 7 eingeschlagen, so drängt er das Andruckelement nach aussen gegen den Marknagel 3. Somit wird eine kraftschlüssige Verbindung zwischen Bolzen und Marknagel hergestellt. Das Einschlagen des Stifts kann auch mit dem bereits beschriebenen pneumatischen Einschlagwerkzeug geschehen.

Zum Entfernen des Bolzens wird zuerst der Stift 8 herausgezogen. Dies kann z.B. mit einer geeigneten Zange geschehen, mit welcher sich der Kopf 11 des Stifts 8 ergreifen lässt. Mit dem Entfernen des Stifts 8 löst sich die Kraft auf das Andruckelement, wodurch dieses sich in den Bolzen zurückzieht und den Bolzen frei gibt.

## Patentansprüche

1. Marknagel mit Arretierungsbolzen, wobei der Marknagel (3) quer zu seiner Längsachse verlaufende Löcher (14) zur Aufnahme des mindestens einen Arretierungsbolzens aufweist und wobei der Arretierungsbolzen einen Kopf (1) und einen im wesentlichen glatten Schaft (2) aufweist, dadurch gekennzeichnet, dass der Durchmesser des Schaftes (2) eines im Marknagel (3) aufgenommenen Arretierungsbolzens in mindestens einer Querrichtung in mindestens einem Bereich des Schaftes (2) durch Manipulation von aussen vergrösserbar ist, derart, dass der Arretierungsbolzen im Marknagel (3) befestigbar ist, dass der Arretierungsbolzen eine durch den Kopf (1) und mindestens durch einen Teil des Schaftes (2) gehende Längsöffnung (7) aufweist, und dass der Schaft (2) des Arretierungsbolzens eine Aussparung (10) und ein in die Aussparung eingesetztes Andruckelement (9) aufweist, wobei ein Teil des Andruckelements (9) in die Längsöffnung (7) hineinragt, so dass durch Einbringen eines Stiftes (8) in die Längsöffnung (7) das Andruckelement (9) nach aussen drückbar ist.

2. Marknagel mit Arretierungsbolzen nach Anspruch 1, dadurch gekennzeichnet, dass der Marknagel zum Ableiten des Knochenmarks eine Abflussausformung (5), ein Abflussloch oder einen Abflussschlitz aufweist.

3. Marknagel mit Arretierungsbolzen nach einem der vorangehenden Ansprüche, dadurch gekennzeichnet, dass der Marknagel einen Marknagelkopf (6) aufweist, welcher so ausgeformt ist, dass er im wesentlichen zugfest, stossfest und rotationsfest mit einem Einschlagwerkzeug verbindbar ist.

## Claims

1. Intramedullary nail with locking pin, wherein the intramedullary nail (3) comprises holes (14) extending transversally to its longitudinal axis for receiving the at least one locking pin, and wherein the locking pin comprises a head (1) and a substantially flat shank (2), characterised in that the diameter of the shank (2) of a locking pin received in the intramedullary nail (3) can be increased in at least one transversal direction in at least one section of the shank (2) by manipulation from the outside such that the locking pin can be locked in the intramedullary nail (3), that the locking pin comprises a longitudinal opening (7) extending through the head (1) and at least through a part of the shank (2), and that the shank (2) of the locking pin comprises a recess (10) and a pressure member (9) inserted in the recess, wherein a part of the pressure member (9) extends into the longitudinal opening (7) such that by inserting a peg (8) into the longitudinal opening (7) the pressure member (9) can be pushed outwards.

2. Intramedullary nail with locking pin of claim 1, characterised in that the intramedullary nail comprises a draining recess (5), a draining hole or a draining slit for draining the marrow.

3. Intramedullary nail with locking pin of one of the preceding claims characterised in that the intramedullary nail comprises a nail head (6) which is shaped such that it can be connected for pulling, pushing and rotation to a driving tool.

## Revendications

1. Clou à moelle avec goujon de retenue, le clou (3) présentant des passages (14) transversaux pour recevoir au moins un goujon de retenue lequel comporte une tête (1) et un fût (2) essentiellement lisse, caractérisé en ce que le diamètre du fût (2) d'un goujon d'arrêt inséré dans le clou à moelle (3) peut être agrandi en au moins une direction transversale située en au moins une région du fût (2) de manière à fixer le goujon dans le clou à moelle (3) par une manipulation exécutée à partir de l'extérieur, que le goujon comporte une ouverture longitudinale (7) traversant sa tête (1) et au moins une partie du fût (2), et que le fût (2) du goujon comporte une échancrure (10) et un organe de blocage (9) disposé dans l'échancrure et pénétrant partiellement à l'intérieur de l'ouverture longitudinale (7) de manière à ce que l'on puisse presser l'organe de blocage (9) vers l'extérieur par introduction d'une tige (8) dans l'ouverture longitudinale (7).

2. Clou à moelle avec goujon de retenue selon la revendication 1, caractérisé en ce que le clou comporte un trou ou une rainure d'évacuation (5) pour l'évacuation de la moelle d'os.

3. Clou à moelle avec goujon de retenue selon une des revendications précédentes, caractérisé en ce que le clou comprend une tête de clou (6) conformée de façon à ce que le clou puisse être réuni avec un outil de frappe d'une manière résistant à la poussée, à la traction et à la rotation.
